# EUROPEAN PATENT APPLICATION

(11) **EP 1 110 546 A1**
(43) Date of publication of application: **27.06.2001**
(21) Application number: 00311610.0
(22) Date of filing: 22.12.2000
(51) Int. Cl.: A61K 9/70, A61K 9/00

(54) **Method of preparing a water soluble film**

(30) Priority: 23.12.1999 US 172085; 12.01.2000 US 481178
(71) Applicant: Johnson & Johnson Consumer Companies, Inc., Skillman, New Jersey 08558-9418 (US)
(72) Inventor: Lin, Shun Y., Plainsboro, New Jersey 08536 (US); Patel, Kalpana J., West Windsor, New Jersey 08550 (US); Link, Martin, Sarasota, Florida 34241 (US); Kang, Maria L., Belle Mead, New Jersey 08502 (US)
(74) Representative: Fisher, Adrian John

(57) **Abstract**

The present invention provides a method of preparing a water soluble film. The method comprises (a) preparing a solution comprising a film former, a water soluble plasticizer, a pharmaceutically active agent, and a solvent; (b) drying the solution at a temperature of from about 50 to about 100° C to form a film; and (c) curing the film at a temperature of from about 15 to about 60° C and at a relative humidity of at least about 30%. The film former is a polyacrylic acid, cellulose derivatives, polyethylene oxide, polyvinyl alcohol, or any combination of any of the foregoing. The water soluble plasticizer contains at least one of a hydroxyl, amido, or amino group and has a boiling point greater than about 150° C. The water soluble film of the present invention may be incorporated into vaginal devices, such as tampons and applicators. This method produces a uniform and homogeneous film which is more flexible and drips less than prior water soluble films, especially those incorporated into vaginal dosage forms. As a result, the film is less irritating. Furthermore, unlike most prior art water soluble films, the film may be shaped to provide a larger contact area within a body cavity, such as the vagina, in order to increase drug delivery. The film is also non-messy.

## Description

This application claims priority from U.S. Serial No. 60/172,085, filed December 23, 1999, which is incorporated herein by reference.

### FIELD OF THE INVENTION

The present invention relates to a method of preparing a water soluble film for use in dosage unit forms, such as tampons and applicators.

### BACKGROUND OF THE INVENTION

Current vaginal dosage forms, except the sponge and film, are messy to use and readily drip out of the vagina. Furthermore, the sponge requires removal after use and is believed to cause infection. Films often cause irritation due to their rigidity and sharp edges.

U.S. Patent Nos. 5,393,528 and 5,529,782 disclose a device having a dissolvable element for administration of an agent material in an internal body area. The dissolvable element is a film made of polyvinyl alcohol, polyethylene oxide, and/or a complex carbohydrate material.

### SUMMARY OF THE INVENTION

The present invention provides a method of preparing a water soluble film. The method comprises the steps of (a) preparing a solution comprising a film former, a water soluble plasticizer, a pharmaceutically active agent, and a solvent; (b) drying the solution at a temperature of from about 50 to about 100° C to form a film; and (c) curing the film at a temperature of from about 15 to about 60° C and a relative humidity of at least about 30%. The film former is a polyacrylic acid, cellulose derivative, polyethylene oxide, polyvinyl alcohol, or any combination of any of the foregoing. The water soluble plasticizer contains at least one of a hydroxyl, amido, or amino group and has a boiling point greater than about 150° C. The water soluble film of the present invention may be incorporated into vaginal devices, such as tampons and applicators. The formulation of the film may be optimized as known in the art to provide controlled release of the pharmaceutically active agent.

This method produces a uniform and homogeneous film which is more flexible and drips less than prior water soluble films, especially those incorporated into vaginal dosage forms. As a result, the film is less irritating. Furthermore, unlike most prior art water soluble films, the film may be shaped to provide a larger contact area within a body cavity, such as the vagina, in order to increase drug delivery. The film is also non-messy.

Another embodiment of the present invention is a dosage unit form, such as a tampon or applicator, comprising a water soluble film prepared by the aforementioned method.

### DETAILED DESCRIPTION OF THE INVENTION

The method of the present invention comprises the steps of (a) preparing a solution comprising a film former, a water soluble plasticizer, a pharmaceutically active agent, and a solvent; (b) drying the solution at a temperature of from about 50 to about 100° C to form a film; and (c) curing the film at a temperature of from about 15 to about 60° C and at a relative humidity of at least about 30%. The inventors have discovered that curing the film under the aforementioned conditions produces a significantly more flexible film which drips less when administered into the vagina and other body cavities than the same film prepared without curing. The film is also non-messy, uniform, and homogeneous.

The solution may be prepared by mixing the ingredients, if the pharmaceutically active agent is water soluble.

Water insoluble pharmaceutically active agents may be dispersed, preferably uniformly, in the solvent by any method known in the art. The other ingredients may be added before or after dispersing the pharmaceutically active agent.

The film former is a polyacrylic acid, cellulose derivative, polyethylene oxide, polyvinyl alcohol, or any combination of any of the foregoing. Suitable cellulose derivatives include, but are not limited to, hydroxyethyl cellulose, carboxymethyl cellulose, hydroxypropyl cellulose, hydroxypropylmethyl cellulose, and any combination of any of the foregoing. The film former is preferably polyvinyl alcohol. More preferably, the film former is a partially hydrogenated polyvinyl alcohol, such as Elvanol™ grade 51-05, 52-22, and 50-42 available from DuPont Co. of Wilmington, DE, and Airvol™ grade 205S and 523S available from Air Products & Chemicals, Inc., of Allentown, PA. The viscosity of the polyvinyl alcohol generally ranges from about 3 to about 1000 cps and preferably ranges from about 3 to about 50 cps. The solution typically comprises from about 5 to about 40% by weight and preferably from about 15 to about 35% by weight of film former, based upon 100% total weight of solution.

The water soluble plasticizer contains at least one of a hydroxyl, amido, or amino group and has a boiling point greater than about 150° C. Preferably, the boiling point of the plasticizer is greater than about 180° C. Suitable plasticizers include, but are not limited to, polyhydroxy compounds, such as propylene glycol, polyethylene glycol, glycerin, and any combination of any of the foregoing. Other suitable plasticizers include, but are not limited to, fatty acid derivatives having a melting point less than about 45 ° C, such as ehydrogenated vegetable oil available as Wecobee™ from Stepan Company of Northfield, IL, and hydrogenated coco-glycerides available as Witepsol H15™ from Hüls America of Somerset, N.J.; and fatty alcohol derivatives having a hydroxy value of greater than about 30. The solution typically comprises from about 0.1 to about 10% by weight and preferably from about 0.5 to about 5% by weight of water soluble plasticizer, based upon 100% total weight of solution.

The pharmaceutically active agent may be water-insoluble or water soluble. Suitable pharmaceutically active agents include, but are not limited to, imidazole antifungal agents, such as imidazole antifungal agents include, but are not limited to, miconazole, econazole, terconazole, ketoconazole, saperconazole, itraconazole, clotrimazole, tioconazole, and butaconazole; antibacterial agents, such as nystatin, neomycin, polymycin, tetracycline, clindamycin, and metronidazole; antiseptic agents, such as oxyquinoline benzoate and aminacrine; hormones, such as estrogens, testolactone, androgens, progestins, megestrol acetate, medroxyprogesterone acetate, esterified estrogens, conjugated estrogens, estradiol, polyestradiol, ethinyl estradiol, estropipate, diethylstilbestrol diphosphate, polyestradiol phosphate, and leuprolide acetate; anti-inflammatory agents, hydrocortisone, triamcinolone, betamethasone, flucinonide, and halcinonide; anesthetics, such as lidocaine and benzocaine; spermicides, such as nonoxynol-9 and octoxynol-9; and any combination of any of the foregoing. A preferred imidazole antifungal agent is miconazole nitrate. A preferred antibacterial agent is metronidazole. A preferred spermicide is nonoxynol-9.

Generally, the amount of pharmaceutically active agent in the solution is an amount effective to accomplish the purpose for which it is being used. The amount of pharmaceutically active agent is typically a pharmaceutically effective amount. However, the amount can be less than a pharmaceutically effective amount when the film is used in a dosage unit form, because the dosage unit form may contain a multiplicity of films or may contain a divided pharmaceutically effective amount. The total effective amount can then be determined in cumulative units containing, in total, a pharmaceutically effective amount of pharmaceutically active agent. The total amount of pharmaceutically active agent may be determined by those skilled in the art. Generally, the solution comprises from about 1 to about 30% by weight and preferably from about 5 to about 20% by weight of pharmaceutically active agent, based upon 100% total weight of solution.

The solvent may be water, ethanol, glycerin, ethylene glycol, amides, amines, or any combination of any of the foregoing. The solvent is preferably water or a mixture of water and ethanol. Preferably, the mixture comprises less than about 30% by weight of ethanol, based upon 100% total weight of mixture. The solution typically comprises from about 20 to about 90% by weight and preferably from about 40 to about 80% by weight of solvent, based upon 100% total weight of solution.

According to a preferred embodiment of the present invention, the solution comprises about 26.4% by weight of polyvinyl alcohol, about 2.4% by weight of glycerin, about 11.2% by weight of nonoxynol 9, and about 60% by weight of water, based upon 100% total weight of solution.

The solution may include other adjuvants, such as surfactants, preservatives, viscosity enhancers, colorants, fragrances, flavorants, lubricants, fillers, binders, wetting agents, penetration agents, pH adjusters, disintegrants, excipients, or any combination of any of the foregoing. Suitable surfactants include, but are not limited to, polyethylene glycol ether of cetearyl alcohol, such as ceteareth-20; hydrogenated coco-glycerides; and any combination of any of the foregoing.

The solution typically has a viscosity of from about 15,000 to about 30,000 cps at room temperature prior to drying. Generally, the water soluble film prepared by the method of the present invention has a thickness of from about 0.03 to about 0.50 mm. Preferably, the thickness of the film is from about 0.05 to about 0.10 mm.

The drying step is generally performed at a temperature of from about 50 to about 100° C. Preferably, the drying step is performed in two stages. In the first stage, the solution is heated to from about 50 to about 70° C. The solution in the first stage is typically heated for less than about 5 minutes. The solution is then heated to from about 70 to about 100° C during the second stage. The solution in the second stage is typically heated for less than about 25 minutes.

The curing step is preferably performed immediately after the drying step. Curing is generally performed at a temperature of from about 15 to about 60° C and at a relative humidity of at least about 30%. Preferably, the curing step is performed at a temperature of from about 25 to about 60° C. The curing step is preferably performed at a relative humidity of at least about 50% and more preferably at a relative humidity of from about 60 to about 90%. The solution may be dried and cured with a drying tunnel having multiple zones or chambers, such as a 5, 6, or 7 zone drying tunnel.

A preferred water soluble film prepared by the method of the present invention comprises about 66% by weight of polyvinyl alcohol, about 6% by weight of glycerin, and about 28% by weight of nonoxynol 9, based upon 100% total weight of water soluble film.

The water soluble film may be coated or laminated onto a substrate, such as non-woven fiber or cotton, by pouring or casting the solution onto the substrate and then drying and curing the solution as described above. Casting may be performed by any method known in the art, such as with a weigh boat, stainless steel tray, teflon rod, cone shape rod, and reverse roller.

The water soluble film alone or coated or laminated on a substrate may be incorporated into a dosage unit form for administration into a body cavity, such as the vagina, rectum, and mouth. The dosage unit form may be a tampon or an applicator. For example, the film coated on a substrate may be utilized as a liner for a tampon. The dosage unit form is preferably flexible. The dosage unit form may be any shape, such as a flat sheet or thimble shape. Preferably, the film is contoured to maximize its contact area with the body cavity for which it is intended to be administered.

According to one embodiment, the outer wrap of the tampon is comprised of non-woven fiber laminiated with the water soluble film. According to another embodiment, the water soluble film is positioned between the inside material of a tampon, such as cotton, and an outer wrap, such as a non-woven fiber material.

A dosage unit form of the present invention containing an antifungal agent, such as miconazole, may be administered to treat yeast infections. It is possible to treat a yeast infection in 3 days, instead of the common 5 day period, with a dosage unit form of the present invention, since a film prepared by the present method has very little drip and may have controlled release of the antifungal agent.

The film may be formulated to be puncture resistant and tear resistant. Also, the film may be formulated to achieve desired release rates of the pharmaceutically active agent as known in the art.

The following examples are intended to describe the present invention without limitation.

### Examples 1-32

Water soluble films having the formulations of Table 1 were prepared as follows. Water was heated to 50-80° C. The film former, *i.e.,* polyvinyl alcohol, is added to the water with constant mixing. The active ingredient, *i.e.,* nonoxynol-9, was added to the solution with constant mixing. The solution was mixed, deaerated, and cooled to room temperature. The solution was coated onto a substrate in the casting device indicated in Table 1 below. The substrate for Examples 1-8 was polypropylene. The substrate for Examples 9-18 and 32 was stainless steel. The substrate for Examples 19-25 was polyester. The substrate for Examples 26-28 was teflon. The substrate for Example 29 was a polyester liner. The substrate for Example 30 was aclar with foil liner. The substrate for Example 31 was a polyethylene and paper liner.

The solution was dried in a multi-zone drying tunnel to form a film. In Examples 1-28 and 31, the solution was dried at a temperature of about 60-90° C for less than about 30 minutes. In Examples 29 and 30, the solution was first dried at a temperature of about 60-75° C for less than about 8 minutes and then dried at a temperature of about 75-90° C for less than about 15 minutes. In Example 32, the solution was first dried at a temperature of about 60-80° C for less than about 5 minutes and then dried at a temperature of about 70-90° C for less than about 25 minutes.

After drying, the film was cured with moisture at a relative humidity of about 30-60% and at about room temperature. For examples 29 and 32, the film was cured with moisture at a relative humidity of about 60-90% and at a temperature of about 40-60° C.

The thickness of the film was measured. The results are shown in Table 1 below.

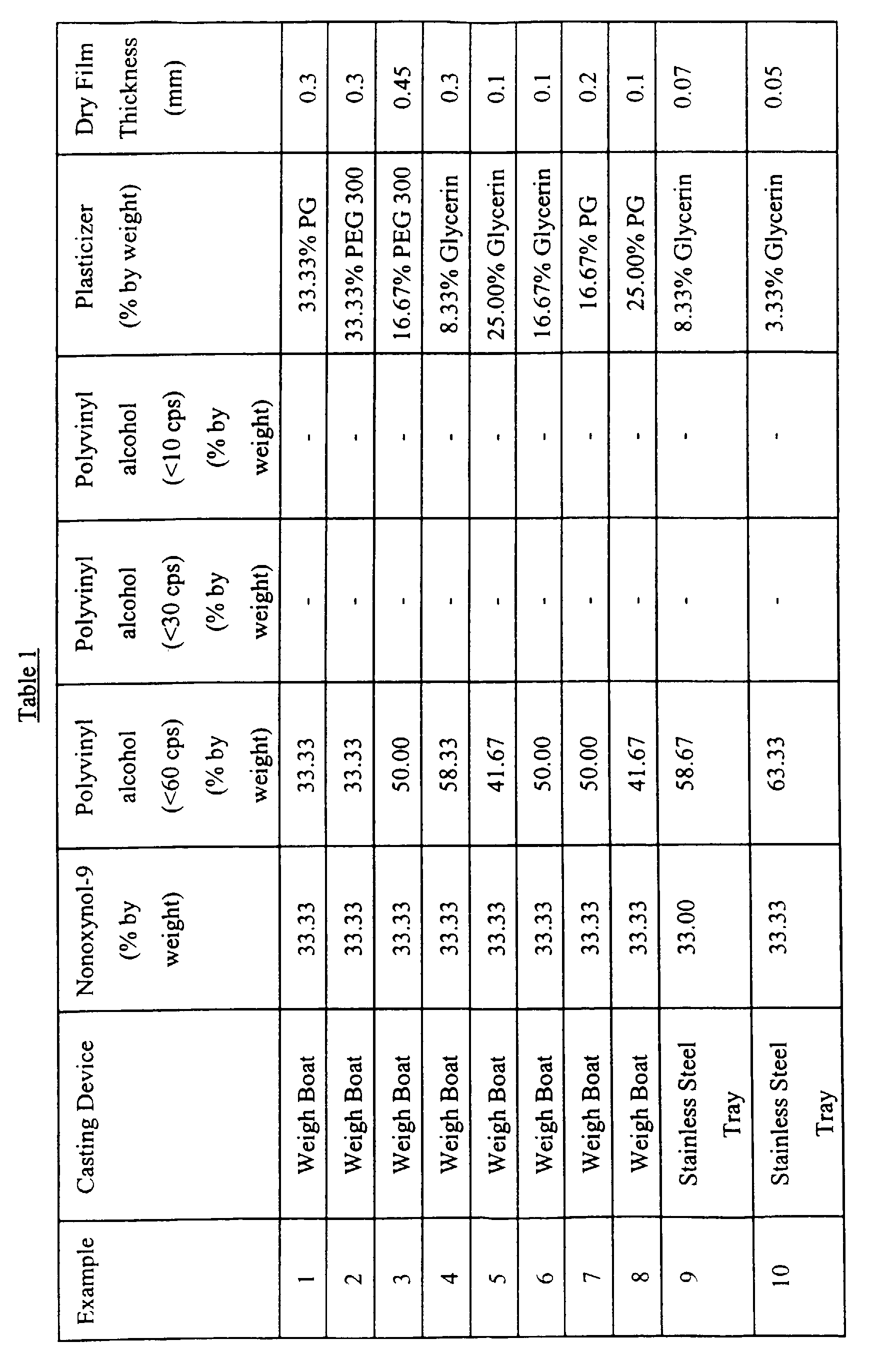

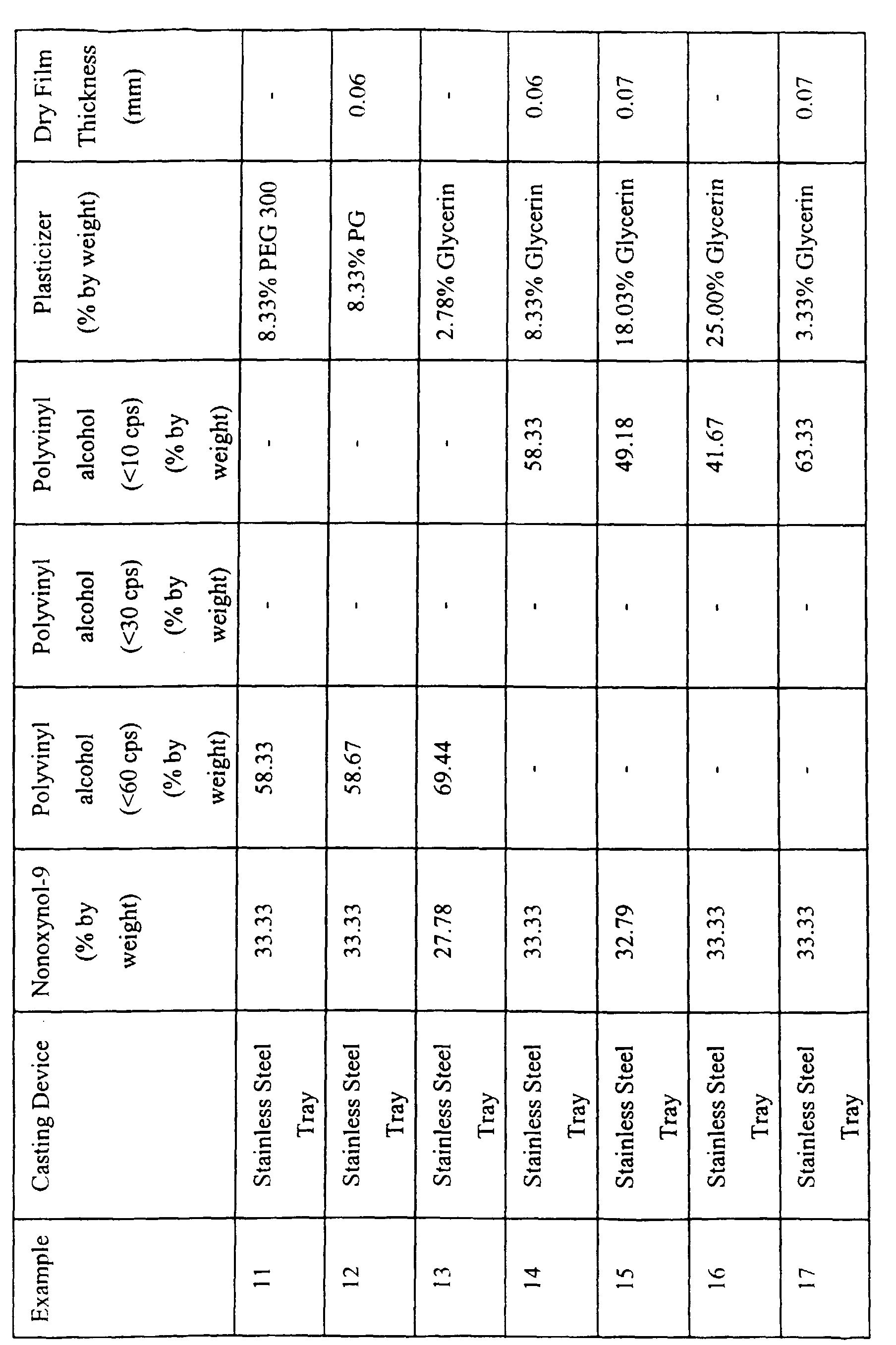

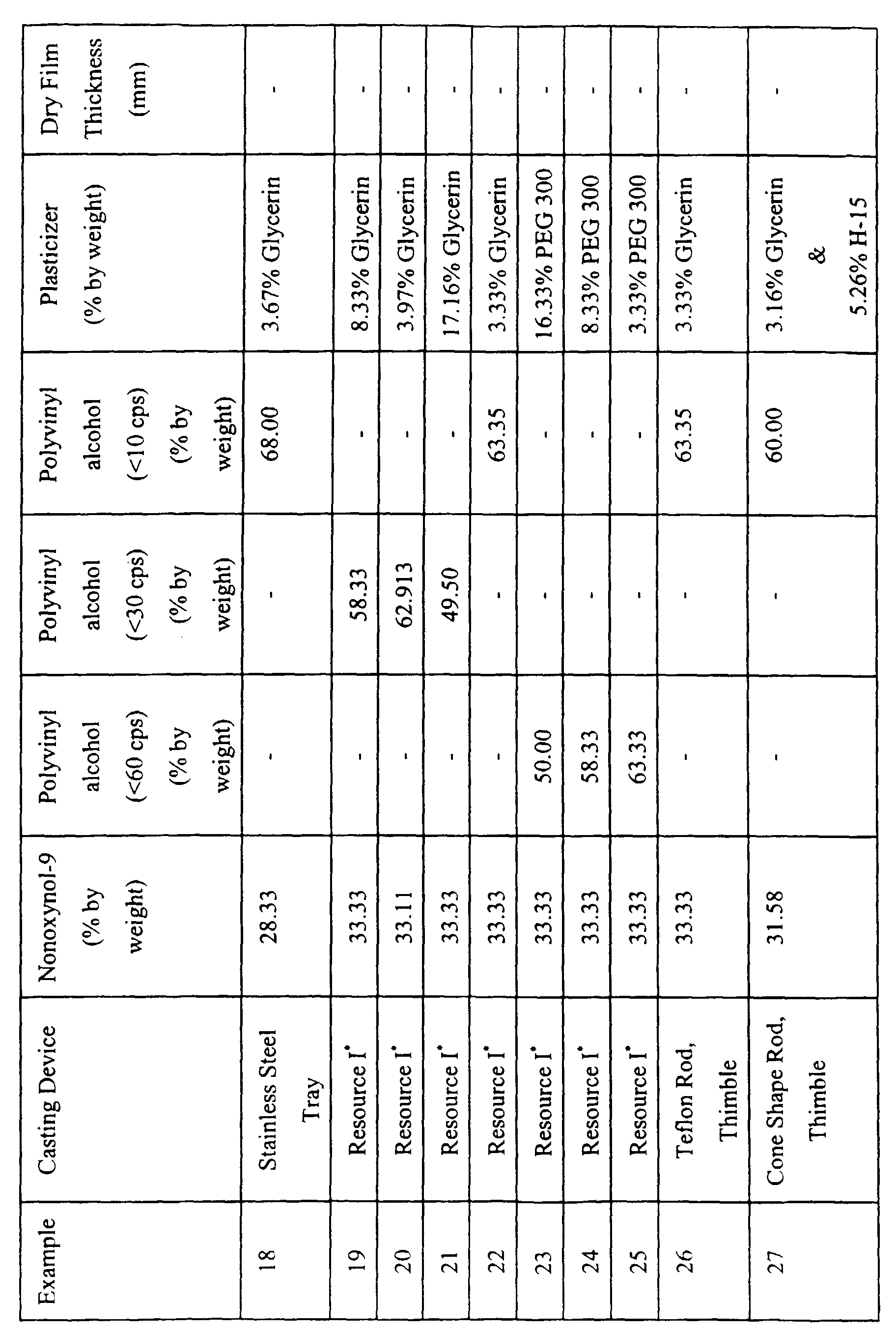

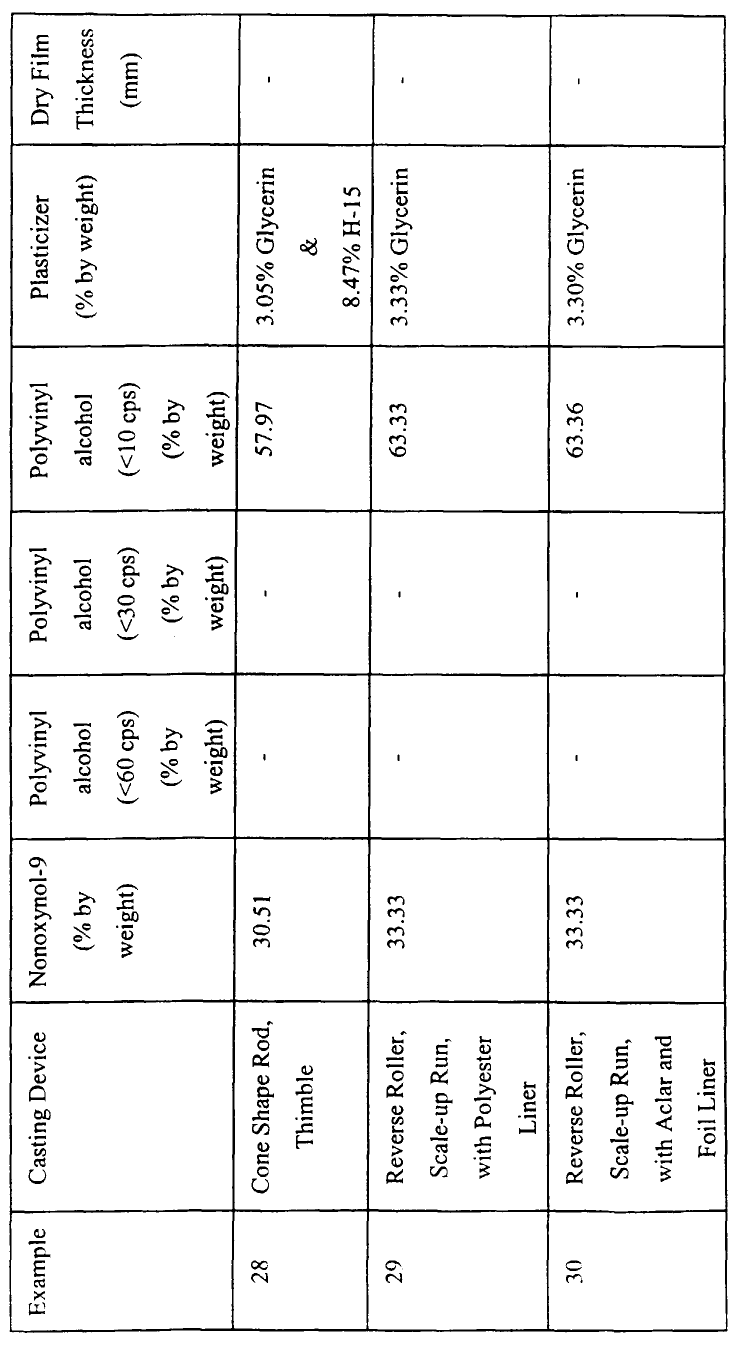

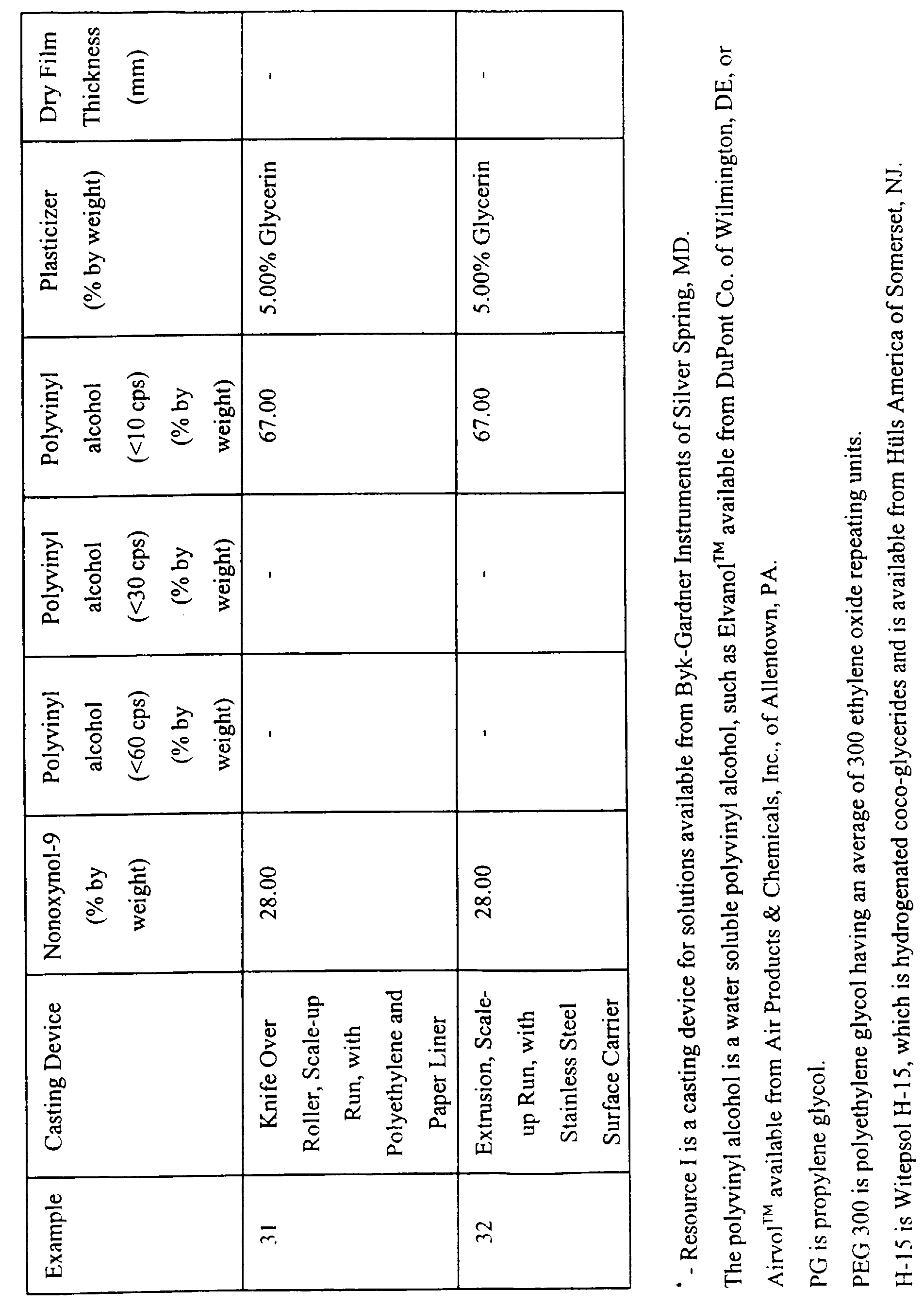

The release rate of nonoxynol-9 from the films prepared and VCF® available from Apothecus Pharmaceutical Corp. of Oyster Bay, NY, in a citrate phosphate buffer having a pH of 4.0 was determined by the USP basket method (United States Pharmacopeia Method Section <711>). The results are shown in Table 2 below. The time to plateau is the time after which there is no significant increase in the release rate.

**Table 2**

| Formulation | Time to Plateau (minutes) | Release Rate (% by weight per minute) |
|---|---|---|
| VCF®¹ | 15-20 | 5.45 |
| Example 6 | 50-60 | 2.59 |
| Example 7 | 50-60 | 3.76 |
| Example 9 | 40-50 | 2.33 |
| Example 10 | 40-50 | 2.97 |
| Example 12 | 40-50 | 3.15 |
| Example 14 | 10-15 | 6.08 |
| Example 15 | 10-15 | 6.66 |
| Example 17 | 10-15 | 6.01 |
| Example 19 | 15-20 | 5.82 |
| Example 20 | 30-40 | 4.33 |
| Example 21 | 30-40 | 3.93 |
| Example 22 | 10-15 | 6.10 |
| Example 23 | 40-50 | 2.34 |
| Example 24 | 30-40 | 2.72 |
| Example 25 | 30-40 | 2.76 |
| Example 26 | 10-15 | 7.23 |
| Example 27 | 5-10 | 8.47 |
| Example 28 | 5-10 | 8.89 |
| Example 29 | <15 | >6.0 |
| Example 30 | <15 | >6.0 |
| Example 31 | <15 | >6.0 |
| Example 32 | <15 | >16 |

### Examples 33-42

Water soluble films having the formulations of Table 3 were prepared as described in Examples 1-32. In Examples 33-41, the solution was dried at a temperature of about 60-90° C for less than about 30 minutes. In Example 42, the solution was first dried at a temperature of about 60-75 ° C for less than about 8 minutes and then dried at a temperature of about 75-90° C for less than about 15 minutes. After drying, the film was cured with moisture at a relative humidity of about 30-60% and at about room temperature.

The substrate for Examples 33-35 was polyester. The substrate for Examples 36-41 was polyester and non-woven fiber. The substrate for Example 42 was a fiber and polyester liner.

The release rate of miconazole nitrate from the films prepared in a citrate phosphate buffer having a pH of 4.0 was determined by the USP basket method for Examples 33-35 and by the following modified USP method for Examples 36-38, 40, and 41. A dialysis membrane with known molecular weight cut-off and diameter was used instead of a mesh basket for holding the test samples. The membrane limited the amount of dissolution medium which contacted the release layer or composition. This modified dissolution procedure was designed to mimic a vaginal environment where only limited amounts of a medium are typically in contact with the composition. Each release layer and composition was tested in an aqueous medium and in a buffered aqueous medium, which were maintained at a pH of about 4.

The results are shown in Table 3 below.

**Table 3**

| Example | Casting Device | Polyvinyl alcohol (<30 cps) (% by weight) | Plasticizer (% by weight) | Miconazole Nitrate (% by weight) | Release Rate |
|---|---|---|---|---|---|
| 33 | Resource I | 36.2 | 18.4% Glycerin & 9.2% EB2 | 36.2 | 3.3%/min |
| 34 | Resource I | 40.0 | 19.9% Glycerin | 40.1 | 4.7%/min |
| 35 | Resource I | 38.0 | 19.0% Glycerin & 4.8% EB2 | 38.2 | 4.7%/min |
| 36 | Resource I & Fiber | 36.2 | 18.4% Glycerin & 9.2% EB2 | 36.2 | 3.50%/hr |
| 37 | Resource I & Fiber | 34.7 | 17.3% Glycerin & 13.2% EB2 | 34.8 | 3.13%/hr |
| 38 | Resource I & Fiber | 38.0 | 19.0% Glycerin & 5.0% EB2 | 38.0 | 3.33%/hr |
| 39 | Resource I & Fiber | 39.6 | 20.6% Glycerin | 39.8 | - |
| 40 | Resource I, Fiber, & OB Tampon | 34.7 | 17.3% Glycerin & 13.2% EB2 | 34.8 | 0.81%/hr |
| 41 | Resource I, Fiber, & OB Tampon | 38.0 | 19.0% Glycerin & 5.0% EB2 | 38.0 | 1.07%/hr |
| 42 | Reverse Roller, Scale-up Run, Fiber & Polyester Liner | 38.1 | 19.1% Glycerin & 4.7% EB2 | 38.1 | - |
| EB2 is Eumulgin B2, which is ceteareth-20 and is available from Henkel Corp. of Hoboken, NJ. | | | | | |
| The polyvinyl alcohol is a water soluble polyvinyl alcohol, such as Elvanol™ available from DuPont Co. of Wilmington, DE, or Airvol™ available from Air Products & Chemicals, Inc., of Allentown, PA. | | | | | |

### Examples 43-46

Water soluble films having the formulations of Table 4 were prepared as described in Examples 1-32. In Examples 43-46, the solution was dried at a temperature of about 60-90 ° C for less than about 30 minutes. After drying, the film was cured with moisture at a relative humidity of about 30-60% and at about room temperature. The substrate for Example 43-46 was polyester.

The time for the dissolution rate to plateau was determined as discussed above.

The results are shown in Table 4 below.

**Table 4**

| Example | Casting Device | Polymer (% by weight) | Plasticizer (% by weight) | Metro-nidazole (% by weight) | Dissolution (Time to Plateau) (min) |
|---|---|---|---|---|---|
| 43 | Resource I | 67.2% PVA 52-22 | 21.7% PEG 400 | 11.1 | 20-30 |
| 44 | Resource I | 58.22% PVA 52-22 | 18.9% PG & 15.7% EB2 | 7.2 | 20-30 |
| 45 | Resource I | 34.9% PVA 52-22 and 11.7% PVA 71-30 | 20.9% PG & 17.5% EB2 | 15.0 | 10-15 |
| 46 | Resource I | 46.6% HPMC E50LV | 20.9% PG & 17.5% EB2 | 15.0 | 5-10 |
| PG is propylene glycol | | | | | |
| PEG is polyethylene glycol. | | | | | |
| EB2 is Eumulgin B2, which is ceteareth-20 and is available from Henkel Corp. of Hoboken, NJ. | | | | | |
| PVA is a water soluble polyvinyl alcohol, such as Elvanol™ available from DuPont Co. of Wilmington, DE, or Airvol™ available from Air Products & Chemicals, Inc., of Allentown, PA. | | | | | |
| HPMC is hydroxypropyl methylcellulose. | | | | | |

### Example 47

A water soluble film having the formulation of Table 5 was prepared as follows. Glycerin and nonoxynol-9 were added into cold water and mixed until uniform. The solution was heated to about 60-80° C and the film former, *i.e.,* polyvinyl alcohol, was added under constant mixing. The solution was mixed, deaerated, and cooled to about room temperature. The solution was coated onto a stainless steel surface with a web thickness of 0.01 to 0.03 cm. The solution was dried in a multi-zone drying tunnel at a temperature of about 60-90° C for less than about 30 minutes to form a film. The film was then cured with moisture at a relative humidity of about 65-90% and at a temperature of about 40-60° C.

**Table 5**

| Ingredient | % by weight |
|---|---|
| Polyvinyl Alcohol (5 cps) | 66.0 |
| Glycerin | 6.0 |
| Nonoxynol-9 | 28.0 |

All patents, publications, applications, and test methods mentioned above are hereby incorporated by reference. Many variations of the present matter will suggest themselves to those skilled in the art in light of the above detailed description. All such obvious variations are within the patented scope of the appended claims.

## Claims

1. A method of preparing a water soluble film, the method comprising the steps of:
(a) preparing a solution comprising:
(i) a film former selected from the group consisting of polyacrylic acids, cellulose derivatives, polyethylene oxide, polyvinyl alcohol, and any combination of any of the foregoing,
(ii) a water soluble plasticizer having at least one of a hydroxyl, amido, or amino group and a boiling point greater than about 150° C,
(iii) a pharmaceutically active agent, and
(iv) a solvent;
(b) drying the solution at a temperature of from about 50 to about 100° C to form a film; and
(c) curing the film at a temperature of from about 15 to about 60° C and at a relative humidity of at least about 30%.

2. The method of claim 1, wherein the solution has a viscosity of from about 15,000 to about 30,000 cps at room temperature prior to drying.

3. The method of claim 1, wherein the film former is polyvinyl alcohol.

4. The method of claim 1, wherein the film former is a partially hydrogenated polyvinyl alcohol.

5. The method of claim 1, wherein the plasticizer is a polyhydroxy compound.

6. The method of claim 5, wherein the plasticizer is selected from the group consisting of propylene glycol, polyethylene glycol, glycerin, and any combination of any of the foregoing.

7. The method of claim 1, wherein the pharmaceutically active agent is selected from imidazole antifungal agents, antibacterial agents, antiseptic agents, hormones, anti-inflammatory agents, anesthetics, spermicides, and any combination of any of the foregoing.

8. The method of claim 1, wherein the pharmaceutically active agent is nonoxynol-9.

9. The method of claim 1, wherein the pharmaceutically active agent is miconazole.

10. The method of claim 1, wherein the water soluble film further comprises
(i) a surfactant,
(ii) a preservative,
(iii) a viscosity enhancer,
(iv) a colorant,
(v) a fragrance,
(vi) a flavorant,
(vii) a lubricant,
(viii) a filler,
(ix) a binder,
(x) a wetting agent,
(xi) a penetration agent,
(xii) a pH adjuster,
(xiii) a disintegrant,
(xiv) an excipient, or
(xv) any combination of any of the foregoing.
